# EUROPÄISCHE PATENTSCHRIFT

(11) **EP 0 810 988 B1**
(45) Veröffentlichungstag und Bekanntmachung des Hinweises auf die Patenterteilung: **13.10.1999**
(21) Anmeldenummer: 96904071.6
(22) Anmeldetag: 13.02.1996
(51) Int. Cl.: C07C 67/36, C07C 69/06

(54) **VERFAHREN ZUR KONTINUIERLICHEN HERSTELLUNG VON METHYLFORMIAT**
METHYL FORMIATE CONTINUOUS PRODUCTION PROCESS
PROCEDE DE PREPARATION EN CONTINU DE FORMIATE DE METHYLE

(30) Priorität: 24.02.1995 DE 19506555
(43) Veröffentlichungstag der Anmeldung: 10.12.1997
(73) Patentinhaber: BASF AKTIENGESELLSCHAFT, 67056 Ludwigshafen (DE)
(72) Erfinder: LIPPERT, Ferdinand, D-67098 Bad Dürkheim (DE); HÖHN, Arthur, D-67281 Kirchheim (DE); DAHLHAUS, Jürgen, D-67117 Limburgerhof (DE)
(86) Internationale Anmeldenummer: EP9600598
(87) Internationale Veröffentlichungsnummer: WO9626178

(56) Entgegenhaltungen:
- DE-B- 1 147 214

## Beschreibung

Die vorliegende Erfindung betrifft ein verbessertes kontinuierliches Verfahren zur Herstellung von Methylformiat durch Umsetzung von Kohlenmonoxid und Methanol unter erhöhtem Druck und erhöhter Temperatur in Gegenwart eines Alkalimetallmethylat als Katalysator.

Das Prinzip dieser Herstellweise für Methylformiat ist lange bekannt.

Methylformiat wird in technischem Maßstab zu Ameisensäure verarbeitet. Dazu wird Methylformiat vor einer Hydrolysestufe destillativ vom Katalysator und höhersiedenden Nebenprodukten getrennt. Der bei der Destillation anfallende Destillationssumpf kann nicht in die Reaktion zurückgeführt werden. Er enthält im Falle der Verwendung von Natriummethylat als Katalysator Natriumhydroxid, Natriumformiat, Natriumcarbonat und Natriumoxalat sowie weitere Verbindungen. Diese Salze müssen in kontinuierlich betriebenen Anlagen entsorgt werden, wodurch beträchtliche Kosten wegen der im Destillationssumpf enthaltenen organischen Verbindungen anfallen (Gehalt an Total Organic Carbon TOC), die in einer Kläranlage abgebaut werden müssen.

Die DE-A 926 785 lehrt ein Verfahren zur Herstellung von Methylformiat aus Methanol und Kohlenmonoxid in Gegenwart von maximal 0,25 Gew.-% Natrium, entsprechend 0,59 Gew.-% Natriummethylat, bezogen auf eingesetztes Methanol. Die Schrift offenbart einen Reaktionsdruck von 300 bar. Die unter diesen Bedingungen erzielbaren Raum-Zeit-Ausbeuten sind jedoch unbefriedigend. Nachteilig ist weiterhin, daß das anfallende Reaktionsgemisch zur Wärmeabführung außerhalb des Reaktors im Kreis gepumpt werden muß.

Die DE-A 11 47 214 lehrt die Aufspaltung des Kohlenmonoxidstroms bei der Herstellung von Methylformiat in zwei Teilströme, die dem Reaktor in verschiedener Höhe zugeführt werden.

Nach der Lehre dieser Schrift werden 0,12 bis 0,3 mol-% Katalysator verwendet, entsprechend über 0,2 Gew.-% Natrium- bzw. 0,26 Gew.-% Kaliummethylat, bezogen auf eingesetztes Methanol. Der Reaktionsdruck beträgt 150 bis 200 bar. Nach längerer Betriebsdauer bilden sich auch bei dieser Betriebsweise Verkrustungen durch Zersetzungsprodukte des Katalysators an Wärmetauschern und Reaktorwänden.

Die DE-A 43 09 731 betrifft eine Verfahrensweise, derzufolge Methanol und Kohlenmonoxid in einer Mischzone teilweise umgesetzt werden, das so erhaltene Gemisch mit Kohlenmonoxid gesättigt wird und die Umsetzung dann in einer Nachreaktionszone ohne weitere Zufuhr von Ausgangsverbindungen zu Ende geführt wird. Der Reaktionsdruck ist bevorzugt ein für Niederdruckverfahren typischer Druck von 40 bis 100 bar. Die Alkalimetallmethylatkonzentration liegt vorzugsweise bei 0,4 bis 1,5 Gew.-%, bezogen auf eingesetztes Methanol.

Die beschriebenen Reaktionsweisen führen zu dem oben beschriebenen Anfall an Destillationssumpf, der eine kostenintensive Entsorgung verlangt.

Es bestand daher die Aufgabe, die Entsorgungskosten für den genannten Destillationssumpf möglichst niedrig zu halten. Insbesondere sollte ein Weg gefunden werden, den Anfall organischer Salze in diesem Destillationssumpf zu vermindern.

Demgemäß wurde ein kontinuierliches Verfahren zur Herstellung von Methylformiat durch Umsetzung von Methanol und Kohlenmonoxid unter erhöhtem Druck und erhöhter Temperatur in Gegenwart von Alkalimetallmethylat gefunden, das dadurch gekennzeichnet ist, daß man die Umsetzung bei einem Druck von 210 bis 250 bar in Gegenwart von 0,05 bis 0,2 Gew.-% Alkalimetallmethylat, bezogen auf eingesetztes Methanol, vornimmt.

Für das erfindungsgemäße Verfahren wird Kohlenmonoxid eingesetzt. Dieses kann mit inerten Gasen wie Stickstoff vermischt werden. Der Gehalt des Gemisches aus Kohlenmonoxid und inerten Gasen an Kohlenmonoxid beträgt aber vorzugsweise mindestens 93 Vol.-%. Um die hydrolytische Zersetzung des Katalysators gering zu halten, sollte der Wassergehalt des Gases weniger als 100 ppm betragen.

Als Katalysator dient ein Alkalimetallmethylat wie Natriummethylat. Bevorzugt wird Kaliummethylat. Der Katalysator wird in einer Menge von 0,05 bis 0,2 Gew.-%, bezogen auf eingesetztes Methanol, eingesetzt. Der Katalysator wird zweckmäßigerweise in Methanol gelöst in die Reaktionszone gegeben.

Kohlenmonoxid, Methanol und Katalysator werden in der Reaktionszone vermischt, wobei eine gute Dispergierung des Gases eine schnelle Umsetzung ermöglicht. Beispielsweise kann das Gas durch eine Strahldüse in den Reaktor gegeben werden. Bevorzugt werden Methanol und der Katalysator im Gegenstrom zum Kohlenmonoxid geführt. Der Kohlenmonoxidstrom kann dabei in zwei Teilströme aufgespalten werden, wie es in der DE-A 11 47 214 beschrieben ist.

Der Reaktionsdruck bei dem erfindungsgemäßen Verfahren beträgt 210 bis 250 bar. Bei geringerem Druck ist die Raum-Zeit-Ausbeute für eine wirtschaftliche Betriebsweise ungenügend, darüber steigt der technische Aufwand zur Druckhaltung unverhältnismäßig an. Bevorzugt ist ein Druck von 215 bis 230 bar. Die Temperatur kann 50 bis 150°C, vorzugsweise 60 bis 110°C betragen

Die molaren Verhältnisse der Ausgangsstoffe Methanol und Kohlenmonoxid können in weiten Grenzen variieren, beispielsweise 2:1 bis 0,5:1, vorzugsweise 1,5:1 bis 1:1.

Die Reaktion kann in Reaktoren wie senkrecht stehenden Reaktionsgefäßen, aber auch in Rohrreaktoren ablaufen. Senkrecht stehende Reaktoren mit innenliegenden Kühlsystemen oder Mantelkühlern sind besonders vorteilhaft. Die Reaktion kann in einem, vorteilhaft aber in einer Kaskade von hintereinander geschalteten Reaktionsgefäßen vorgenommen werden. Diese Kaskade kann ein Temperaturprofil aufweisen mit der höchsten Temperatur im ersten und der niedrigsten im letzten Reaktionsgefäß.

Über die Verweilzeit der Reaktanden im Reaktor läßt sich der Umsatz der Reaktion steuern. Hohe Endumsätze verringern die Mengen von nach der Aufarbeitung in die Reaktion zurückzuführenden Ausgangsstoffen. Bewährt haben sich CO-Umsätze von 85 bis 99 %.

Die erfindungsgemäß erhaltene Reaktionslösung kann in an sich bekannter Weise aufgearbeitet werden. In der Regel schließt sich dabei an eine Entspannung und Restgasabtrennung eine Destillation der flüssigen Bestandteile an, wobei das so erhaltene Methanol in die Reaktion zurückgeführt werden kann. Methylformiat kann in bekannter Weise zu Ameisensäure hydrolysiert werden.

Durch das erfindungsgemäße Verfahren kann bei niedrigen Katalysatorkonzentrationen, die die Einsatzstoffkosten deutlich erniedrigen, eine hohe Raum-Zeit-Ausbeute von über 800 g/l·h bei Methylformiat-Endkonzentration von über 97 Gew.-% (vor Aufarbeitung) erzielt werden.

Der Anfall an organischen Salzen im Destillationssumpf geht beträchtlich zurück und erlaubt so eine kostengünstigere Entsorgung.

Weiterhin erlaubt die Verwendung von Kaliummethylat als Katalysator eine salzfreie Fahrweise, d.h. es kommt zu keinen Salzablagerungen an Wärmetauschern, Rohren oder Ventilen, wenn der während der Reaktion erzielte Gehalt der Reaktionslösung an Methylformiat auf maximal 95 Gew.-% begrenzt wird.

Darüberhinaus wurde unerwarteterweise festgestellt, daß die Ausübung des erfindungsgemäßen Verfahrens im technischen Maßstab dazu führt, daß aufgrund der im Vergleich zum Stand der Technik wesentlich seltener auftretenden Verkrustungen von Wärmetauschern, Rohrleitungen und Ventilen die für ihre Beseitigung notwendigen Abstellzeiten der Anlage erheblich reduziert werden konnten. Das führt umgekehrt zu einer höheren Verfügbarkeit der Anlage und somit zu einer höheren Jahreskapazität.

### Beispiele

### Beispiele 1 bis 6

Vier seriell geschaltete Rohrreaktoren (2 m x 45 mm, V = 3,15 l) wurden von unten nach oben betrieben. Kohlenmonoxid wurde über eine Strahldüse in den ersten Reaktor dosiert. Die Reaktoren hatten ein innenliegendes Wärmetauscherrohr. Die Ausgangsverbindungen konnten in jeden Reaktor einzeln dosiert werden. Das Reaktionsgemisch konnte nach jedem einzelnen Reaktor entnommen werden. Der Katalysator wurde in Methanol gelöst in Reaktor 1 (R1) dosiert, Methanol wurde in R1 gegeben. Der Reaktionsdruck betrug 220 bar.

Die folgende Tabelle gibt die wesentlichen Daten der Reaktion wieder. CO wurde entweder in R1 oder sowohl in R1 wie auch in Reaktor 2 (R2) gegeben.

Der Austrag wurde gaschromatographisch und naßanalytisch auf den Gehalt an Methylformiat analysiert. Der Umsatz ist bezogen auf eingesetztes Methanol. RZA steht für Raum-Zeit-Ausbeute, bezogen auf das Gesamtvolumen aller jeweils eingesetzten Reaktoren. NaOMe steht für Natriummethylat, KOMe für Kaliummethylat und MeFo für Methylformiat. Die nach der Destillation anfallende Salzmenge war gegenüber Versuchen mit höheren Katalysatorkonzentrationen deutlich reduziert.

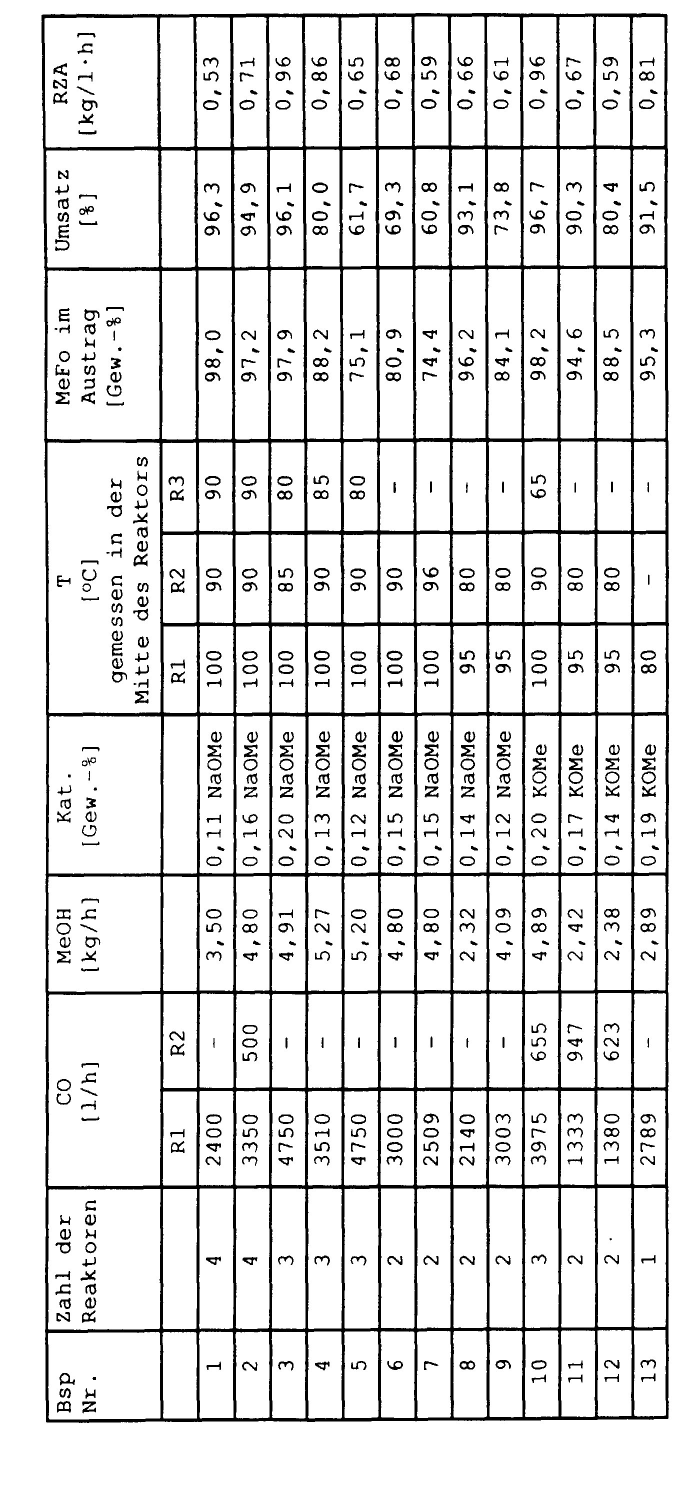

## Patentansprüche

1. Verfahren zur kontinuierlichen Herstellung von Methylformiat durch Umsetzung von Kohlenmonoxid und Methanol unter erhöhtem Druck und erhöhter Temperatur in Gegenwart eines Alkalimetallmethylats, dadurch gekennzeichnet, daß man die Umsetzung bei einem Druck von 210 bis 250 bar in Gegenwart von 0,05 bis 0,2 Gew.-% Alkalimetallmethylat, bezogen auf eingesetztes Methanol, vornimmt.

2. Verfahren nach Anspruch 1, dadurch gekennzeichnet, daß man bei einem Druck von 215 bis 230 bar arbeitet.

3. Verfahren nach Anspruch 1 oder 2, dadurch gekennzeichnet, daß man bei einer Temperatur von 50 bis 150°C arbeitet.

4. Verfahren nach den Ansprüchen 1 bis 3, dadurch gekennzeichnet, daß man Kaliummethylat als Katalysator verwendet.

## Claims

1. A process for the continuous preparation of methyl formate by reaction of carbon monoxide and methanol under elevated pressure and elevated temperature in the presence of an alkali metal methoxide, wherein the reaction is carried out at a pressure of from 210 to 250 bar in the presence of from 0.05 to 0.2% by weight of alkali metal methoxide, based on methanol used.

2. A process as claimed in claim 1, carried out at a pressure of from 215 to 230 bar.

3. A process as claimed in claim 1 or 2, carried out at from 50 to 150°C.

4. A process as claimed in any of claims 1 to 3, wherein potassium methoxide is used as catalyst.

## Revendications

1. Procédé de préparation en continu de formiate de méthyle par réaction de monoxyde de carbone et de méthanol sous pression élevée et à température élevée en présence d'un méthylate de métal alcalin, caractérisé en ce que l'on mène la réaction sous une pression de 210-250 bar en présence de 0,05-0,2% en poids d'un méthylate de métal alcalin, par rapport au méthanol introduit.

2. Procédé selon la revendication 1, caractérisé en ce que l'on travaille sous une pression de 215-230 bar.

3. Procédé selon la revendication 1 ou 2, caractérisé en ce que l'on travaille à une température de 50-150°C.

4. Procédé selon l'une quelconque des revendications 1 à 3, caractérisé en ce que l'on utilise du méthylate de potassium en tant que catalyseur.
